# FASCICULE DE BREVET EUROPEEN

(11) **EP 2 809 351 B1**
(45) Date de publication et mention de la délivrance du brevet: **19.07.2017**
(21) Numéro de dépôt: 13706584.3
(22) Date de dépôt: 29.01.2013
(51) Int. Cl.: A61K 47/26, A61K 9/00, A61K 31/07, A61K 31/355, A61K 31/375, A61K 31/4415, A61K 31/455, A61K 31/525

(54) **PROCÉDÉ DE PRÉPARATION D'UN PRODUIT SIRUPEUX COMPRENANT DES VITAMINES**
VERFAHREN ZUR HERSTELLUNG EINES EIN SIRUPPRODUKTS MIT VITAMINEN
METHOD FOR PREPARING A SYRUPY PRODUCT COMPRISING VITAMINS

(30) Priorité: 30.01.2012 WO PCT/FR2012/050197
(43) Date de publication de la demande: 10.12.2014
(73) Titulaire: URGO RECHERCHE INNOVATION ET DEVELOPPEMENT, 21300 Chenôve (FR)
(72) Inventeur: EYMARD, Franck, F-21240 Talant (FR); GABIOT, Pierre, F-21000 Dijon (FR); BRENON, Romain, F-21410 Gissey Sur Ouche (FR); LAMOISE, Michel, F-21110 Bessey les Citeaux (FR)
(74) Mandataire: Cabinet Plasseraud
(86) Numéro de dépôt international: PCT/FR2013/050179
(87) Numéro de publication internationale: WO 2013/114034

(56) Documents cités:
- EP-A2- 1 320 356
- US-A1- 2010 092 446
- US-B1- 6 211 169

## Description

La présente invention se rapporte à un procédé de préparation d'un produit sirupeux comprenant des vitamines. La plupart, voire la totalité des étapes du procédé sont réalisées sous atmosphère inerte. De plus, le produit sirupeux est entièrement désoxygéné avant son conditionnement afin d'avoir un taux d'oxygène dissout dans le produit sirupeux inexistant, ce qui permet d'éviter la dégradation des vitamines lors de la préparation du produit sirupeux, lors de son stockage une fois conditionné, et même après une première consommation du produit par l'utilisateur.

Une alimentation saine et équilibrée est supposée permettre de satisfaire aux apports journaliers recommandés (AJR) en matière de vitamines et minéraux. Toutefois, le mode d'alimentation moderne peut parfois induire certaines carences alimentaires.

Les vitamines, en dose allant du microgramme à plusieurs milligrammes par jour, sont nécessaires au métabolisme des organismes vivants et notamment celui de l'homme. L'organisme ne peut cependant généralement pas les synthétiser dans les quantités recommandées pour satisfaire aux besoins journaliers. Chez l'être humain, seules trois vitamines sont synthétisées par des bactéries intestinales : les vitamines K, B8 et B12.

Un apport insuffisant ou une absence de vitamine provoque respectivement une hypovitaminose ou une avitaminose qui peuvent être la cause de diverses maladies (scorbut, béribéri, rachitisme, etc) .

De plus, les vitamines jouent un rôle primordial dans la croissance et le développement de l'enfant. Ainsi, les vitamines liposolubles (telles que les vitamines A, E ou K), et plus préférentiellement la vitamine D, se révèlent indispensables à l'enfant et à l'adolescent.

La vitamine D joue un rôle essentiel dans l'absorption intestinale du calcium et la minéralisation du squelette. Ce besoin en vitamine D peut se révéler nécessaire pour l'enfant surtout quand celui-ci vit dans des régions présentant un ensoleillement faible, et ce encore plus pendant l'hiver.

Du côté des vitamines hydrosolubles, les vitamines C et B1 sont particulièrement importantes pour le jeune enfant comme pour l'adolescent ou l'adulte.

Il est donc souvent nécessaire de fournir, par le biais de compléments alimentaires, différentes associations de vitamines sous une forme concentrée de manière à compléter le régime alimentaire d'un individu.

La formulation des compléments alimentaires vitaminés se heurte cependant au problème de la grande instabilité des vitamines vis-à-vis de l'oxygène, de la température, du pH ou encore de la lumière.

Les compléments alimentaires vitaminés sont ainsi le plus souvent formulés sous forme de gélules pour que les vitamines ne se trouvent pas au contact direct de l'oxygène de l'air, telles que les gélules commercialisées par la société Nycomed® sous la dénomination Dynatonic®.

Il est cependant souhaitable de proposer des formulations vitaminées sous forme liquide, notamment de produit sirupeux, plus facile à administrer, surtout dans le cas des enfants.

Des produits sirupeux comprenant des vitamines ont déjà été proposés. Cependant, pour compenser la dégradation des vitamines au contact de l'oxygène, il a jusqu'ici été nécessaire d'introduire les vitamines en une quantité initiale plus importante que celle qui se retrouve effectivement dans le produit final, c'est-à-dire, d'employer la technique du surdosage initial en produits vitaminiques afin d'obtenir un produit sirupeux présentant un ratio vitaminique individuel et/ou global proche de 100%.

Par ratio vitaminique individuel, on entend, au sens de la présente invention, le ratio de la quantité d'une vitamine introduite sur la quantité de ladite vitamine retrouvée en fin de procédé. Il est calculé individuellement pour chacune des vitamines introduites dans le procédé selon l'invention.

Par ratio vitaminique global, on entend, au sens de la présente invention, le ratio de la quantité introduite de toutes les vitamines sur la quantité desdites vitamines retrouvées en fin de procédé. Ce ratio est calculé à partir de toutes les vitamines introduites dans le procédé selon l'invention.

De plus, la dégradation des vitamines a tendance à se poursuivre pendant la durée de conservation du produit sirupeux au sein de son conditionnement. Le surdosage des vitamines et la durée de vie limitée du produit engendrent un surcoût important pour les produits vitaminés sous forme liquide, ce qui les rend peu rentables au niveau industriel.

Le brevet EP 1 320 356 décrit un procédé de préparation d'un sirop comprenant des vitamines ayant une longue durée de vie. Le sirop se présente sous la forme d'une émulsion huile-dans-eau réalisée en mélangeant une phase aqueuse contenant des vitamines hydrosolubles, un agent gélifiant et un épaississant, avec une phase huile contenant les vitamines liposolubles et un tensioactif. Les vitamines liposolubles se trouvent à l'intérieur des particules d'huile dispersées dans la phase continue aqueuse et sont ainsi protégées vis-à-vis de l'oxydation par l'air. Lorsque le sirop contient également des vitamines hydrosolubles, celles-ci se trouvent à l'intérieur de gouttelettes d'eau comprises dans les particules d'huile dispersées dans la phase aqueuse, ce qui résulte en une émulsion eau-dans-huile-dans-eau qui peut poser des problèmes de stabilité au stockage lors du procédé de fabrication mais aussi après conditionnement dudit produit, et ce encore plus après une première utilisation du produit par le consommateur. Dans cette demande, le problème de pertes vitaminiques dès l'initiation du procédé de fabrication du sirop n'est résolu que par un surdosage en vitamines en début du procédé et ce, afin d'obtenir un ratio vitaminique global avoisinant les 100% en fin de procédé. Ainsi, aucune solution au problème de surdosage en matières premières instables dans un procédé de fabrication d'un produit sirupeux de qualité pharmaceutique n'est amenée par ce document.

Il existe donc un réel besoin dans la mise au point d'un nouveau procédé de fabrication d'un produit sirupeux comprenant des vitamines et présentant une bonne stabilité au stockage sans surdosage de la quantité de vitamines au moment de leur introduction dans le procédé et permettant d'obtenir un ratio vitaminique individuel et/ou global optimal en fin de procédé.

La demanderesse a trouvé qu'il était possible d'obtenir un procédé simple et économique de fabrication d'un produit sirupeux permettant d'optimiser la quantité de vitamines introduites en tant que matières premières en prévenant mais aussi en évitant leur dégradation au cours de la préparation et dans le produit fini conditionné.

L'invention a donc pour objet un procédé de préparation d'un produit sirupeux comprenant, dans un milieu pharmaceutiquement acceptable, des vitamines, ledit procédé comprenant les étapes suivantes :
a) l'inertage de tous les appareillages mis en oeuvre dans le procédé
b) l'introduction dans lesdits appareillages, d'un sirop de sucre et/ou d'un dérivé de sucre choisi parmi les fibres solubles à base de dextrine et les polyols et l'introduction des vitamines sous atmosphère inerte ;
c) l'ajout de glucose déshydraté et désoxygéné sous atmosphère inerte ;
d) la désoxygénation du produit sirupeux ainsi formé ;
e) le conditionnement sous atmosphère inerte du produit sirupeux.

L'invention a également pour objet, selon un autre aspect, un procédé de préparation d'un produit sirupeux comprenant, dans un milieu pharmaceutiquement acceptable, des vitamines, ledit procédé comprenant les étapes suivantes :
a) l'inertage de tous les appareillages mis en oeuvre dans le procédé
b) l'introduction, dans lesdits appareillages, d'un sirop de sucre et/ou d'un dérivé de sucre et l'introduction des vitamines sous atmosphère inerte ;
c) optionnellement l'ajout de glucose déshydraté et désoxygéné sous atmosphère inerte ;
d) la désoxygénation du produit sirupeux ainsi formé ;
e) le conditionnement sous atmosphère inerte du produit sirupeux.

Par milieu pharmaceutiquement acceptable, on entend, au sens de la présente invention, un milieu qui est compatible avec une administration orale du produit sirupeux. Le milieu pharmaceutiquement acceptable peut notamment comprendre de l'eau. Selon un mode de réalisation préféré, le milieu pharmaceutiquement acceptable ne comprend pas d'autre solvant que l'eau, préalablement purifiée et désoxygénée.

Par sirop de sucre, on entend, au sens de la présente invention, une solution de sucre dans de l'eau déminéralisée. Par sucre, on entend tout monosaccharide tel que notamment le glucose, le dextrose, le fructose, le galactose, le mannose, ou tout disaccharide tel que le saccharose (aussi appelé sucrose), le lactose ou le maltose, conférant une saveur sucrée à l'aliment dans lequel ils sont introduits. Dans le sirop de sucre utilisé dans le procédé selon l'invention, la quantité de sucre, notamment de saccharose peut notamment aller de 65 à 70% en poids par rapport au poids total du sirop de sucre, de préférence de 66.5 à 67.5%, et plus préférentiellement de l'ordre de 67%.

Par «dérivé de sucre», on entend toute substance synthétique ou naturelle dont le pouvoir sucrant est comparable ou plus élevé que celui du saccharose. Le dérivé de sucre peut ainsi être un édulcorant présentant, par rapport au sucre, un pouvoir cariogène faible, une valeur calorique réduite et un bénéfice sur la santé pour les sujets diabétiques.

Par produit sirupeux, on entend, au sens de la présente demande, un produit à base de sirop de sucre et/ou de dérivé de sucre, ledit produit sirupeux pouvant par ailleurs comprendre tout autre composé, tel que notamment des vitamines. Le produit sirupeux présente notamment une viscosité supérieure à celle de l'eau, mais reste fluide et peut notamment s'écouler sous son propre poids.

Par vitamines, on entend au sens de la présente invention, des molécules organiques nécessaires à la croissance et au bon fonctionnement de l'organisme des êtres vivants. Les vitamines sont classiquement réparties en deux catégories en fonction de leur solubilité : les vitamines hydrosolubles et les vitamines liposolubles.

Des exemples de vitamines hydrosolubles présentes dans le produit sirupeux préparé selon l'invention sont notamment : la vitamine B1 (thiamine), la vitamine B2 (riboflavine), la vitamine B3 (acide nicotinique) appelée également vitamine PP (Pellagra Preventive), la vitamine B5 (acide pantothénique), la vitamine B6 (pyridoxine), la vitamine B8 ou H (biotine), la vitamine B9 (acide folique), la vitamine B12 (cobalamine) et la vitamine C (acide ascorbique).

Des exemples de vitamines liposolubles présentes dans le produit sirupeux préparé selon l'invention sont notamment : la vitamine A (rétinol), la vitamine D3 (cholécalciférol), la vitamine E (mélange de tocophérols et de tocotriénols) et la vitamine K (phylloquinone).

Par glucose déshydraté on entend, au sens de la présente invention, une poudre de glucose ayant une teneur en eau inférieure à 10%, de préférence inférieure à 5%.

### Figure

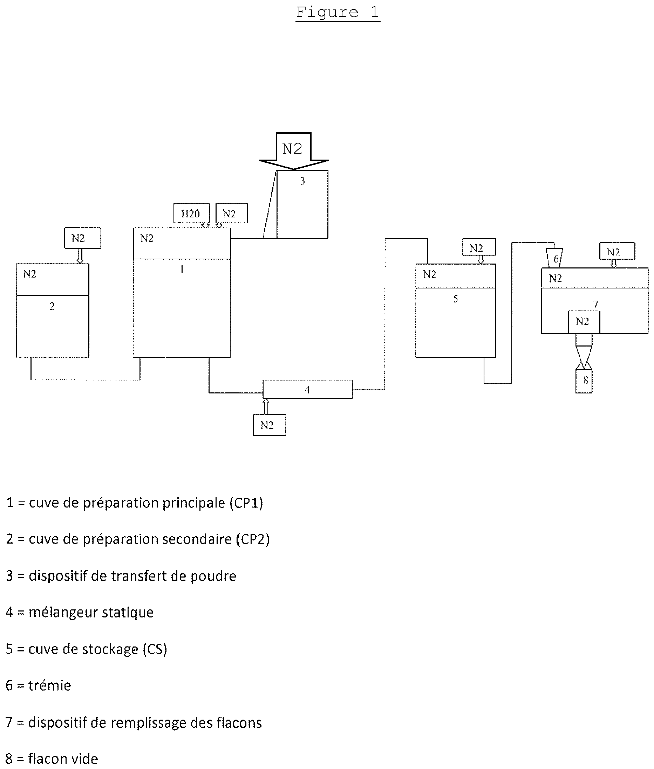

La figure 1 illustre, de manière schématique, le procédé selon la présente invention.

### Inertage des appareillages

Le procédé selon l'invention comprend une étape a) d'inertage préalable de tous les appareillages mis oeuvre dans la préparation du produit sirupeux.

Par «inertage» on entend, au sens de la présente demande, le remplacement de l'air contenu dans un volume par un gaz inerte.

Par «appareillages» on entend, au sens de la présente demande, tous les dispositifs mécaniques mis en oeuvre dans la réalisation du procédé selon la présente invention, tel que notamment les différentes cuves, le dispositif de transfert de poudre, le mélangeur statique, la trémie située en amont du dispositif de conditionnement, le dispositif de conditionnement ou encore les canalisations.

Il est à noter que, par la suite, l'inertage des appareillages peut, de préférence, être maintenu grâce à un bullage continu réalisé dans chacun desdits appareillages.

L'étape d'inertage des appareillages mis en oeuvre dans le procédé selon l'invention tels que les cuves, le dispositif de transfert de poudre, le mélangeur statique, le dispositif de conditionnement et les canalisations permet notamment d'éviter la dégradation des vitamines pendant le procédé.

L'inertage peut notamment être réalisé en injectant une surpression de gaz inerte dans les cuves, le dispositif de transfert de poudre, le mélangeur statique, le dispositif de conditionnement et les canalisations pendant une durée suffisante pour obtenir un taux d'oxygène acceptable.

Par taux d'oxygène acceptable, on entend, au sens de la présente invention, un taux d'oxygène compris entre 0 et 4% en volume d'oxygène par volume de produit, de préférence entre 0 et 2% en volume et plus préférentiellement inférieur à 0.1% en volume.

Le taux d'oxygène peut être contrôlé notamment dans les cuves ou les canalisations par une sonde placée dans lesdites cuves et canalisations.

En particulier, le taux d'oxygène est mesuré avec une sonde METTLER InPro 6850i, selon les paramètres standards fournis par le fabricant.

L'injection de gaz inerte dans les appareillages, notamment dans les cuves et les canalisations, peut notamment être réalisée à une pression allant de 0,1 à 12 bars, de préférence de 0,2 à 5 bars, et plus préférentiellement encore de l'ordre de 0,45 bars.

L'injection de gaz inerte dans les appareillages, notamment dans les cuves et les canalisations peut notamment être réalisée pendant une durée allant de 100 à 1000 secondes, de préférence de 200 à 800 secondes, et plus préférentiellement de 300 à 600 secondes.

Le gaz inerte, exempt d'oxygène, injecté, peut notamment être de l'azote ou du dioxyde de carbone, de préférence le gaz inerte est l'azote.

### Introduction du sirop de sucre et/ou d'un dérivé de sucre et des vitamines

Le procédé selon l'invention comprend une étape b) d'introduction du sirop de sucre et/ou d'un dérivé de sucre choisi parmi les fibres solubles à base de dextrine et les polyols et l'introduction des vitamines. Selon un mode préféré de réalisation, le sirop de sucre est un sirop à base de saccharose.

Par fibres solubles à base de dextrine, on entend au sens de la présente invention tout résidu partiellement hydrolysé issu d'un procédé de chauffage en présence d'un composé acide de qualité alimentaire, de l'amidon de blé ou de maïs, et se présentant sous forme de fibres solubles. Ces fibres se trouvent introduites dans le procédé objet de l'invention sous forme de poudre. On peut par exemple retrouver ce composé commercialisé sous la dénomination de Nutriose® FB par la société Roquette.

Les polyols peuvent être choisis de manière non limitative parmi le maltitol, le mannitol, le sorbitol, le xylitol ou l'isomalt.

De préférence, le polyol est le maltitol, par exemple commercialisé par la société Roquette sous la marque Lycasin®.

Selon un autre mode préféré de réalisation, le polyol peut être le sorbitol. Le sorbitol peut être introduit dans le procédé objet de la présente invention sous forme de poudre et présente en outre de bonnes propriétés humectante et stabilisante en plus de son pouvoir sucrant. On le retrouve par exemple commercialisé sous la dénomination Neosorb® par la société Roquette.

Lorsque le polyol est introduit sous forme de poudre, du sirop de sucre est de préférence introduit avec le polyol pour obtenir le produit final sirupeux.

La quantité de fibres solubles à base de dextrine, ou de polyols peut notamment être comprise entre 5 et 20% par rapport au poids total du produit sirupeux final obtenu. Préférentiellement, la quantité de fibres solubles à base de dextrine, ou de polyols est comprise entre 7 et 16% par rapport au poids total du produit sirupeux final obtenu.

L'introduction du sirop de sucre et/ou du dérivé de sucre et des vitamines est réalisée sous atmosphère inerte afin de limiter la quantité d'oxygène dissout dans le mélange comprenant le sirop de sucre et les vitamines.

Par introduction sous atmosphère inerte, on entend, au sens de la présente invention, que les appareillages, notamment la cuve dans laquelle sont introduits les constituants du produit sirupeux, est munie d'une entrée de gaz inerte en fond de cuve, au moyen par exemple d'une canne plongeante en inox fritté, ce qui permet de réaliser un bullage de gaz inerte dans le contenu de la cuve.

Selon un mode de réalisation préféré, le bullage peut notamment être effectué avec un débit allant de 0,1 à 10 Nm³/h, de préférence de 2 à 8 Nm³/h, et plus préférentiellement encore de l'ordre de 4,5 Nm³/h.

Selon un autre mode de réalisation préféré, le bullage peut notamment être réalisé avec une pression allant de 0,1 à 1 bars, de préférence de 0,3 à 0,7 7 bars et plus préférentiellement encore de l'ordre de 0,45 bars.

On introduit tout d'abord le sirop de sucre et/ou le dérivé de sucre dans la cuve principale de préparation.

Selon un mode de réalisation préféré le sirop de sucre est introduit via le haut de la cuve et le dérivé de sucre est introduit par aspiration en fond de cuve. Dans ce dernier cas, la cuve est avantageusement munie d'une pompe à vide qui permet de faire le vide dans la cuve et d'aspirer le dérivé de sucre.

Afin d'abaisser encore le taux d'oxygène dissout dans le sirop de sucre et/ou le dérivé de sucre, on peut préférentiellement maintenir le sucre et/ou le dérivé de sucre sous agitation et bullage d'un gaz inerte pendant une durée allant de 1 à 6 heures, de préférence de 2 à 5h, et plus préférentiellement de 4h.

Selon un mode de réalisation préféré, le sirop de sucre et/ou le dérivé de sucre peut être chauffé à une température ne dépassant notamment pas les 50°C. En effet, à une température excédant les 50°C, les vitamines liposolubles subiraient une dégradation immédiate lors de leur introduction dans le sirop de sucre et/ou le dérivé de sucre.

Le pH du sirop de sucre et/ou du dérivé de sucre peut, de préférence, être ajusté entre 8 et 10, plus préférentiellement entre 8,5 et 9,5, et notamment aux environs de 9, par exemple en introduisant une base telle que l'hydroxyde de sodium. L'ajustement du pH permet d'une part la stabilisation et la protection du produit sirupeux intermédiaire formé à cette étape, mais aussi la préservation des vitamines liposolubles introduites dans le sirop sucre de sucre et/ou le dérivé de sucre.

En effet, ces vitamines liposolubles doivent se trouver dans un milieu basique au moment de leur introduction. Dans le cas contraire, leur dégradation est favorisée.

Les vitamines sont donc ensuite introduites dans le sirop de sucre et/ou le dérivé de sucre.

Selon un mode de réalisation préféré, on réalise une introduction séquentielle des vitamines dans le sirop de sucre et/ou le dérivé de sucre, c'est-à-dire que l'on introduit en premier les vitamines liposolubles puis on introduit ensuite les vitamines hydrosolubles.

Selon un autre mode de réalisation préféré, les vitamines liposolubles sont préalablement dispersées avec un tensioactif, tel que notamment un tensioactif huile-dans-eau, d'origine chimique, ou naturelle tel qu'un monoglycéride, un diglycéride, un sucroester, un phospholipide ou encore un polysaccharide, comme par exemple le galactomannane, la pectine, la caséine, la gomme arabique ou encore la gélatine, avant leur introduction dans le sirop de sucre et/ou le dérivé de sucre. Cette opération permet de faciliter l'émulsification des vitamines liposolubles avec le sirop de sucre. En effet, le tensioactif possède un rôle d'initiateur dans la création de l'émulsion vitaminique globale.

Il est à noter que cette préparation favorisera également l'homogénéisation des vitamines liposolubles avec les vitamines hydrosolubles, une fois ces dernières ajoutées au mélange.

Comme décrit précédemment, les vitamines sont sensibles à la chaleur et limiter la température de chauffage permet d'éviter leur dégradation pendant le procédé. Ainsi, un chauffage de la pré-émulsion à une température inférieure à 50°C, mais supérieure à 35°C, permet une bonne homogénéisation du mélange sans dégrader les vitamines liposolubles.

Afin de ne perdre aucune des vitamines introduites, on peut notamment procéder au rinçage de la cuve contenant la préémulsion après son transfert dans le sirop de sucre et/ou le dérivé de sucre. Le rinçage peut notamment être effectué avec de l'eau, de préférence de l'eau purifiée et désoxygénée, et ce toujours à une température ne dépassant pas les 50°C.

Une fois que le mélange sirop de sucre et/ou dérivé de sucre et vitamines liposolubles est homogène, on peut procéder à l'introduction des vitamines hydrosolubles.

La température du mélange dans lequel on introduit les vitamines hydrosolubles peut également être abaissée à une température ne dépassant pas 35°C. Cette faible température de chauffage permet de ne pas dégrader les vitamines hydrosolubles sensibles à la chaleur, notamment au-delà des 35°C.

Afin de ne pas perdre de vitamines, les réservoirs contenant les vitamines peuvent être rincés, notamment avec de l'eau, de préférence avec de l'eau désoxygénée et purifiée, et même chauffée, mais à une température ne dépassant pas les 35°C.

Selon un mode très particulier de réalisation de la présente invention, on peut également envisager d'introduire les vitamines dans le procédé sous la forme d'un pré-mélange des vitamines liposolubles et hydrosolubles. Ce pré-mélange possède avantageusement toutes les propriétés de l'introduction séquentielle des vitamines, à savoir notamment une absence de dégradation de ces dernières et une capacité à s'émulsionner de bonne facture. Ce pré-mélange est commercialisé par la société Vitablend, par exemple.

Selon un mode particulier de réalisation, on peut également introduire, dans le mélange comprenant le sirop de sucre et/ou le dérivé de sucre et les vitamines, un arôme, tel que notamment un arôme de prune, afin d'améliorer le goût du produit sirupeux comprenant des vitamines. L'arôme peut notamment être introduit dans le sirop en même temps que les vitamines hydrosolubles.

### Ajout de glucose déshydraté et désoxygéné

Le procédé selon l'invention peut comprendre une étape c) d'ajout de glucose déshydraté.

Le glucose déshydraté peut notamment être introduit dans la cuve contenant le sirop de sucre et/ou le dérivé de sucre et les vitamines au moyen d'un système de transfert du sucre déshydraté mis sous désoxygénation permanente, c'est-à-dire d'un appareil capable de transférer le glucose déshydraté dans la cuve sans y introduire d'oxygène.

Selon un mode de réalisation préféré, le glucose déshydraté est désoxygéné au moyen d'un appareil comprenant une pompe à vide, ledit appareil permettant ensuite l'introduction du glucose dans la cuve par une surpression de gaz inerte, tel que notamment d'azote.

Un tel appareil est notamment décrit dans le brevet US 6 325 572 et est commercialisé par la société Dietrich Engineering Consultants sous la dénomination PTS®.

Ce type d'appareil fonctionne par la création de vide dans le corps dudit appareil de transfert, puis le remplissage par la poudre d'intérêt, à savoir du glucose déshydraté et désoxygéné. Enfin, en utilisant le différentiel de pression entre l'intérieur de cet appareil mis sous pression d'azote et la cuve du présent procédé auquel il se retrouve rattaché, le glucose déshydraté et désoxygéné est relâché à l'intérieur de la cuve dudit procédé.

### Désoxygénation du produit sirupeux

Une fois que les différents constituants du produit sirupeux préparé par le procédé selon l'invention ont été mélangés, le procédé selon l'invention comprend une étape d) de désoxygénation du produit sirupeux ainsi formé, afin d'obtenir un taux d'oxygène le plus faible possible, de préférence nul, avant l'étape de conditionnement.

L'étape de désoxygénation du produit sirupeux peut notamment être réalisée dans un mélangeur statique, par exemple un mélangeur statique vendu sous la dénomination commerciale SMV® par la société Sulzer.

Par mélangeur statique, on entend, au sens de la présente invention, un réservoir comprenant des hélices à pales inversées, dans lequel on injecte un gaz inerte, soumettant à turbulence le liquide à désoxygéner.

Ces hélices à pales inversées permettent d'augmenter le temps de passage dudit liquide dans le mélangeur statique et assurent un mélange en régime turbulent du liquide mélangé au gaz inerte. Plus les hélices à pales inversées sont nombreuses, plus le régime est turbulent et plus la désoxygénation s'avère efficace.

Selon un mode de réalisation préféré, le mélangeur statique est opéré avec un débit de gaz inerte allant de 0.1 à 20 Nm³/h, de préférence de 1 à 10 Nm³/h, et plus préférentiellement encore de l'ordre de 8,5 Nm³/h.

Selon un autre mode de réalisation préféré, le mélangeur statique est opéré avec une pression de gaz inerte allant de 0.1 à 12 bars, de préférence de 0.2 à 5 bars et plus préférentiellement encore de l'ordre de 0,45 bars.

Selon un autre mode de réalisation préféré, le mélangeur statique présente une longueur de 1 m et un diamètre de 50 cm. En fonction du débit et de la viscosité du liquide injecté dans ce mélangeur, les dimensions du mélangeur statique et le nombre d'hélices à pales inversées peuvent être modifiés en conséquence.

En sortie du mélangeur statique, le liquide est saturé en microbulles de gaz inerte et ne contient plus d'oxygène dissout.

Le taux d'oxygène dissout peut être contrôlé par une sonde à oxygène telle que décrite précédemment.

Le taux d'oxygène dissout dans le produit sirupeux après l'étape de désoxygénation peut notamment être une valeur allant de 0 à 0.4 ppm, de préférence de 0 à 0.2 ppm et plus préférentiellement encore de 0 à 0,1 ppm.

### Conditionnement du produit sirupeux

La dernière étape du procédé selon l'invention, est le conditionnement du produit sirupeux dans des flacons individuels.

Selon un mode préféré de réalisation, le produit sirupeux obtenu par le procédé selon la présente invention est conditionné dans des flacons de type « aérosols ».

Le produit sirupeux peut être acheminé vers les flacons de conditionnement au moyen d'une trémie, reliée au mélangeur statique et auxdits flacons par des canalisations.

Par trémie, on entend, au sens de la présente invention, un entonnoir étanche, de forme conique ayant un rôle de réservoir tampon mécanique, qui permet de réguler le débit du liquide.

Le volume de produit sirupeux introduit dans chaque flacon peut notamment être contrôlé par l'utilisation d'un système de dosage, comme par exemple une pompe volumétrique, un système pondéral, un système optique ou encore un système temps-pression.

L'étape de conditionnement du procédé selon l'invention est réalisée sous atmosphère inerte. Ainsi, les deux canalisations autour de la trémie et la trémie elle-même sont inertées avant le passage du produit sirupeux.

En particulier, les flacons dans lesquels le produit sirupeux est conditionné sont inertés avant leur remplissage par injection d'un gaz inerte.

Selon un mode de réalisation préféré le gaz inerte est injecté avec une pression allant de 0,1 à 5 bars, de préférence de 0,5 à 2 bars et plus préférentiellement encore de l'ordre de 1 bar.

Selon un autre mode de réalisation préféré, le gaz inerte est injecté pendant une durée allant de 10 à 2000 millisecondes, de préférence de 50 à 500 millisecondes et plus préférentiellement encore de l'ordre de 100 millisecondes.

Il est à noter que le procédé selon l'invention permet une conservation optimale du produit sirupeux. En particulier, lorsque le produit est conditionné sous forme d'aérosols, une conservation optimale est maintenue même lorsque le produit a déjà été ouvert une première fois. En effet, la forme aérosol dudit flacon combinée à la présence d'azote retrouvée dans la partie aérienne du flacon, permettent une conservation du produit sirupeux même lorsque l'utilisateur a déjà commencé à consommer une partie dudit produit sirupeux contenu dans ledit flacon. Ce type de flacon peut être désigné sous l'appellation de flacon pressurisé.

A cette étape, il peut être calculé ce que la demanderesse appellera le ratio vitaminique individuel et le ratio vitaminique global.

Selon un mode préféré de réalisation, le ratio vitaminique individuel peut notamment être supérieur à 95%, de préférence supérieur à 98%, et plus préférentiellement supérieur à 98,6%.

Selon un mode plus préféré de réalisation, le ratio vitaminique global peut notamment être supérieur à 98%, de préférence supérieur à 99%, et plus préférentiellement supérieur ou égal à 99,3%. En particulier, l'homme du métier veillera à choisir les vitamines présentant des ratios vitaminiques individuels suffisamment élevés pour permettre d'obtenir le ratio vitaminique global souhaité.

### Stockage intermédiaire du produit sirupeux

Entre l'étape de désoxygénation du produit sirupeux et l'étape de conditionnement, on peut envisager une étape intermédiaire de stockage du produit sirupeux dans une cuve.

Afin de maintenir au minimum le taux d'oxygène dissout dans le produit sirupeux, l'étape intermédiaire de stockage du produit sirupeux dans une cuve est conduite sous atmosphère inerte et le produit sirupeux est maintenu sous agitation.

En particulier, la cuve de stockage peut être inertée avant le transfert du produit sirupeux dans ladite cuve. Une fois le produit sirupeux transféré dans la cuve de stockage, celui-ci peut être maintenu sous atmosphère inerte par bullage d'un gaz inerte.

Selon un mode de réalisation préféré, le bullage est effectué avec un débit allant de 0,1 à 20 Nm³/h, de préférence de 1 à 10 Nm³/h, et plus préférentiellement encore de l'ordre de 2 Nm³/h.

Selon un autre mode de réalisation préféré, le bullage est réalisé avec une pression allant de 0,1 à 12 bars, de préférence de 0,2 à 5 bars et plus préférentiellement encore de l'ordre de 0,45 bars.

Selon un autre mode de réalisation de l'invention, celle-ci peut consister en un flacon pressurisé comprenant un produit sirupeux susceptible d'être obtenu directement par le procédé objet de l'invention.

Selon cet autre mode de réalisation de l'invention, le flacon pressurisé peut être de type aérosol comprenant un produit sirupeux susceptible d'être obtenu directement par le procédé objet de la présente invention et caractérisé en ce que le ratio vitaminique global dudit produit sirupeux est supérieur ou égal à 99,3%.

L'invention est illustrée plus en détails à l'aide des exemples suivants qui sont donnés à titre purement illustratif et non limitatif.

### Exemples:

### Exemple 1 : Eléments du procédé mis en oeuvre

On met en oeuvre un procédé selon la Figure 1 qui comprend les éléments suivants : une cuve de préparation principale (CP1) et une cuve de préparation secondaire (CP2) qui sont reliées par une canalisation. Ces deux cuves de préparation sont utilisées pour réaliser le mélange des différents constituants du produit sirupeux. Ces deux cuves sont chauffées grâce à des doubles enveloppes dans lesquelles, à l'intérieur de cet espace confiné, circule un liquide caloporteur (du type eau ou encore huile). Les deux cuves de préparations sont chacune équipées d'une entrée de gaz inerte afin de réaliser l'inertage des cuves et d'une canne en inox fritté en fond de cuve qui permet de réaliser un bullage de gaz inerte dans le contenu des cuves. Les deux cuves de préparation sont aussi équipées de mobiles d'agitation, du type pale à hélice ou encore turbine, afin de pouvoir mélanger le contenu des cuves. La cuve de préparation principale est également munie d'un appareil de transfert de poudre commercialisé par la société Dietrich Engineering Consultants afin d'introduire le glucose déshydraté dans la cuve sans y faire entrer d'oxygène. La cuve de préparation principale comprend également une entrée d'eau, l'eau étant préalablement purifiée et désoxygénée par un échangeur statique. La cuve de préparation principale est reliée à un mélangeur statique par une canalisation. Le mélangeur statique est vendu sous la dénomination commerciale SMV® par la société SULZER, il a une longueur de 1 m et un diamètre de 50 cm. Il est muni d'une entrée de gaz inerte. Le mélangeur statique est relié à une cuve de stockage (CS) par une canalisation. La cuve de stockage est munie d'une entrée de gaz inerte afin d'inerter la cuve et d'une canne en inox fritté en fond de cuve qui permet de réaliser un bullage de gaz inerte dans le contenu de la cuve. La cuve de stockage est également équipée d'une turbine ou d'une pale à hélice, afin de pouvoir mélanger le contenu de la cuve. La cuve de stockage est reliée au poste de conditionnement par une canalisation. Le poste de conditionnement est constitué d'une trémie et d'une machine de remplissage de flacons qui sont reliées entre elles par une canalisation. Le système de dosage, à savoir une pompe volumétrique, permet de réguler le débit du liquide qui arrive dans la machine de remplissage des flacons. La machine de remplissage des flacons est munie d'un embout qui s'adapte aux goulots des flacons et peut ainsi les inerter, les remplir avec le produit sirupeux comprenant des vitamines et les maintenir sous atmosphère inerte jusqu'à ce qu'ils soient bouchés, mais aussi après ouverture du consommable, notamment lors d'une utilisation quotidienne par le consommateur.

### Exemple 2 : Préparation d'un produit sirupeux par le procédé selon l'invention

Dans le procédé de préparation du produit sirupeux selon l'invention, les quantités de matières premières suivantes sont utilisées :

| **CONSTITUANTS** | **QUANTITES (en Kg)** |
|---|---|
| Sirop de sucre | 2 000 |
| Lessive de soude | 12,5 |
| Acide ascorbique | 3 |
| Montanox 80 | 12,2 |
| Vitamine A | 0,350 |
| Vitamine E | 3,6 |
| Arôme | 0,2 |
| Vitamine B6 | 0,35 |
| Vitamine B3 | 3,2 |
| Vitamine B2 | 0,360 |
| Vitamine B5 | 1,1 |
| Glucose déshydraté | 184 |
| Eau purifiée | qsp. 2 600 |

Le procédé se décompose selon les étapes suivantes :
- on inerte les deux cuves de préparation (CP1 et CP2) et la canalisation les reliant avec une surpression de gaz inerte à 0,4 bars pendant 300 sec. ;
- on introduit le sirop de sucre dans la cuve de préparation principale (CP1) ;
- le sirop de sucre est laissé sous agitation et sous bullage d'azote avec une pression de 0,4 bars et un débit de 4 Nm³/h pendant 4 heures ;
- on chauffe le sirop de sucre à 50°C à l'aide des doubles enveloppes entourant la cuve CP1;
- on ajuste le pH du sirop de sucre à 9,5 en introduisant la lessive de soude et l'acide ascorbique ;
- on préchauffe la cuve de préparation secondaire (CP2) à 50°C à l'aide des doubles enveloppes entourant la cuve CP2;
- on introduit dans la cuve de préparation secondaire (CP2) le Montanox 80 et les vitamines liposolubles suivantes : vitamine A, vitamine D3, vitamine E ;
- on laisse le mélange de la cuve CP2 sous agitation à 50°C et sous bullage d'azote pendant 60 minutes ;
- le contenu de la cuve CP2 est transféré à la cuve CP1 par la canalisation les reliant ;
- on rince plusieurs fois la cuve CP2 avec de l'eau à 50°C préalablement purifiée et désoxygénée et l'eau de rinçage est ensuite transférée dans le cuve CP1 par la canalisation reliant CP2 à CP1 ;
- on laisse le contenu de la cuve CP1 sous agitation à 50°C et sous bullage d'azote pendant 60 minutes ;
- on abaisse la température du contenu de la cuve CP1 à 35°C ;
- on introduit dans la cuve CP1 les vitamines hydrosolubles suivantes : vitamine B1, vitamine B2, vitamine B3, vitamine B5, vitamine B6, vitamine B8, vitamine B12, vitamine C ainsi que l'arôme de prune ;
- on rince les réservoirs qui contenaient les vitamines à l'eau purifiée et l'eau de rinçage est ajoutée dans la cuve CP1 ;
- on laisse le contenu de la cuve CP1 sous agitation à 35°C et sous bullage d'azote pendant 120 minutes ;
- on introduit le glucose déshydraté dans la cuve CP1 par le système de transfert de poudre ;
- on introduit de l'eau purifiée et désoxygénée dans la cuve CP1 ;
- on laisse le contenu de la cuve CP1 sous agitation à 35°C et sous bullage d'azote pendant 60 minutes ;
- on inerte la tuyauterie entre la cuve CP1 et la cuve de stockage, et on inerte la cuve de stockage avec une pression d'azote à 0,4 bars et un débit de 1 Nm³/h pendant 300 secondes ;
- on transfère le contenu de la cuve CP1 à la cuve de stockage en passant par le mélangeur dans lequel on injecte de l'azote à contresens du passage du liquide avec une pression de 0,4 bars et un débit de 8 Nm³/h ;
- le produit sirupeux qui s'accumule progressivement dans la cuve de stockage est maintenu sous agitation à 150 rpm et sous bullage d'azote avec une pression de 0.4 bars et un débit de 2 Nm3/h ;
- les canalisations entre la cuve de stockage et la machine de remplissage des flacons, ainsi que la trémie de conditionnement sont inertées avec de l'azote à une pression de 0,3 bars pendant 600 secondes ;
- le produit sirupeux est transféré vers la trémie de conditionnement et le contenu est maintenu sous une pression d'azote à 0,2 bars ;
- le produit sirupeux est progressivement transféré vers la machine de remplissage des flacons ;
- les flacons sont inertés avec de l'azote à 1 bar pendant 100 millisecondes puis ils sont remplis avec un volume de produit sirupeux dosé par une pompe volumétrique et ils sont maintenus sous atmosphère d'azote jusqu'à ce qu'ils soient fermés par une valve sertie.

On prend au hasard un des flacons produits et on dose les vitamines contenues dans ce flacon. Les rendements sont calculés en divisant la quantité réelle de vitamines qui est contenue dans le flacon par la quantité de vitamines théorique qui devrait se trouver dans le flacon, et en multipliant le résultat par 100. Les rendements sont regroupés dans le tableau suivant :

| **VITAMINES** | **RENDEMENT** |
|---|---|
| Vitamine A | 100% |
| Vitamine B2 | 98,6% |
| Vitamine B3 | 98,8% |
| Vitamine B5 | 100% |
| Vitamine B6 | 98,6% |
| Vitamine E | 100% |
| Ensemble des vitamines | 99,3% |

On remarque que les différents ratios vitaminiques individuels sont très bons puisqu'ils sont compris entre 98,6 et 100%.

Il en est de même pour le ratio vitaminique global, puisque celui-ci est de 99,3%.

Ainsi, les spécifications indiquées sur les flacons pour chaque vitamine sont respectées sans avoir à surdoser les vitamines, permettant d'obtenir ainsi un produit sirupeux vitaminiques de qualité pharmaceutique.

Il est à noter que ce mode de réalisation particulier du procédé, objet de la présente invention, n'est en aucun cas limitatif aux matières premières exemplifiées *supra* pouvant être introduites dans la réalisation dudit produit sirupeux. Ainsi, tout produit pouvant être ingéré par l'être humain, sensible à l'oxygène, ou encore pouvant se présenter sous forme d'émulsion, peut être intégré dans ladite fabrication dudit produit sirupeux, et ce, sans se limiter à une quantité définie. Par exemple, il est envisageable d'introduire en sus divers composés choisis parmi la liste non limitative comprenant les probiotiques, les sels minéraux, les hormones, les acides aminés, certains principes actifs issus des plantes, les alcaloïdes, les agents conservateurs tel que le sorbate de potassium ou encore les antioxydants. Bien entendu, l'introduction de ces différents composés n'a aucun effet sur la qualité du produit sirupeux obtenu.

### Exemple 3 : Préparation d'un produit sirupeux par le procédé selon l'invention

Selon un mode de réalisation similaire au procédé décrit dans l'exemple 2, un produit sirupeux comprenant les quantités de matières premières suivantes est réalisé :

| **CONSTITUANTS** | **QUANTITES (en Kg)** |
|---|---|
| Sirop de sucre | 2 000 |
| Lessive de soude | 12,5 |
| Acide ascorbique | 3 |
| Montanox 80 | 12,2 |
| Vitamine C | 16 |
| Vitamine D3 | 0,001 |
| Vitamine A | 0,350 |
| Vitamine E | 3,6 |
| Arôme | 0,2 |
| Vitamine B1 | 0,247 |
| Vitamine B8 | 0,01 |
| Vitamine B12 | 0,55 |
| Vitamine B6 | 0,35 |
| Vitamine B3 | 3,2 |
| Vitamine B2 | 0,360 |
| Vitamine B5 | 1,1 |
| Glucose déshydraté | 184 |
| Eau purifiée | qsp. 2 600 |

Conformément au dernier paragraphe de l'exemple 2, il est ainsi possible de réaliser toute une variété de produits sirupeux dont les matières premières utilisées ainsi que leurs quantités d'introduction lors de la mise en oeuvre dudit procédé, sont tout à fait variables. En revanche, la qualité du produit sirupeux obtenue en fin de procédé reste la même.

### Exemple 4 : Préparation d'un produit sirupeux par le procédé selon l'invention

Selon un mode de réalisation particulier de l'invention, un produit sirupeux comprenant les quantités de matières premières suivantes est réalisé :

| **CONSTITUANTS** | **QUANTITES (en Kg)** |
|---|---|
| Sirop de sucre | 2 000 |
| Lessive de soude | 12,5 |
| Acide ascorbique | 3 |
| Montanox 80 | 12,2 |
| Vitamine C | 16 |
| Vitamine D3 | 0,001 |
| Vitamine A | 0,350 |
| Vitamine E | 3,6 |
| Arôme | 0,2 |
| Vitamine B1 | 0,247 |
| Vitamine B8 | 0,01 |
| Vitamine B9 | 0,06 |
| Vitamine B6 | 0,35 |
| Vitamine B3 | 3,2 |
| Vitamine B2 | 0,360 |
| Vitamine B5 | 1,1 |
| Sorbate de potassium | 5,09 |
| Sirop de maltitol | 409 |
| Eau purifiée | qsp. 2 600 |

Conformément au dernier paragraphe de l'exemple 2, il est ainsi possible de réaliser toute une variété de produits sirupeux dont les matières premières utilisées ainsi que leurs quantités d'introduction lors de la mise en oeuvre dudit procédé, sont tout à fait variables. En revanche, la qualité du produit sirupeux obtenue en fin de procédé reste la même.

## Revendications

1. Procédé de préparation d'un produit sirupeux comprenant des vitamines, ledit procédé comprenant les étapes suivantes :
a) l'inertage de tous les appareillages mis en oeuvre dans le procédé
b) l'introduction dans lesdits appareillages, d'un sirop de sucre et/ou d'un dérivé de sucre choisi parmi les fibres solubles à base de dextrine et les polyols et l'introduction des vitamines sous atmosphère inerte ;
c) optionnellement l'ajout de glucose déshydraté et désoxygéné sous atmosphère inerte ;
d) la désoxygénation du produit sirupeux ainsi formé ;
e) le conditionnement sous atmosphère inerte du produit sirupeux.

2. Procédé selon la revendication 1, **caractérisé en ce que** l'introduction des vitamines sous atmosphère inerte est réalisée de façon séquentielle.

3. Procédé selon l'une quelconque des revendications précédentes, **caractérisé en ce que** l'étape d'introduction des vitamines comprend en outre l'introduction d'un agent tensioactif d'origine chimique ou naturelle choisi parmi la liste des gélatines, des pectines, de la caséine, des gommes arabiques ou des galactomannanes, ainsi que d'eau préalablement purifiée et désoxygénée.

4. Procédé selon l'une quelconque des revendications précédentes, **caractérisé en ce qu'**il comprend une étape intermédiaire de stockage du produit sirupeux dans une cuve sous atmosphère inerte et sous agitation entre les étapes de désoxygénation et de conditionnement du produit sirupeux.

5. Procédé selon l'une quelconque des revendications précédentes, **caractérisé en ce que** l'inertage de l'atmosphère est réalisé dans les appareillages mis en oeuvre dans le procédé avant l'introduction de quelconque matière première, et est maintenu par bullage d'un gaz inerte avec un débit allant de 0.1 à 20 Nm³/h, de préférence de 2 à 8 Nm³/h, et plus préférentiellement encore de l'ordre de 4,5 Nm³/h, et une pression allant de 0.1 à 1 bars, de préférence de 0.3 à 0.7 bars et plus préférentiellement encore de l'ordre de 0,45 bars.

6. Procédé selon l'une quelconque des revendications précédentes, **caractérisé en ce que** le glucose déshydraté est désoxygéné au moyen d'un système de transfert de sucre déshydraté mis sous désoxygénation permanente, ledit système permettant ensuite l'introduction du glucose dans la cuve.

7. Procédé selon l'une quelconque des revendications précédentes, **caractérisé en ce que** l'étape de désoxygénation du produit sirupeux est réalisée dans un mélangeur statique opéré avec un débit de gaz inerte allant de 0.1 à 20 Nm³/h, de préférence de 1 à 10 Nm³/h, et plus préférentiellement encore de l'ordre de 8 Nm³/h, et une pression allant de 0.1 à 12 bars, de préférence de 0.2 à 5 bars et plus préférentiellement encore de l'ordre de 0,45 bars.

8. Procédé selon l'une quelconque des revendications précédentes, **caractérisé en ce que** le taux d'oxygène dissous dans le produit sirupeux après l'étape de désoxygénation est une valeur allant de 0 à 0.4 ppm, de préférence de 0 à 0.2 ppm et plus préférentiellement encore de 0 à 0,1 ppm.

9. Procédé selon l'une quelconque des revendications précédentes, **caractérisé en ce que** le conditionnement du produit sirupeux est mis en oeuvre au moyen d'un système de dosage.

10. Procédé selon l'une quelconque des revendications précédentes, **caractérisé en ce que** les flacons dans lesquels le produit sirupeux est conditionné sont inertés avant leur remplissage par injection d'un gaz inerte avec une pression allant de 0.1 à 5 bars, de préférence de 0.5 à 2 bars et plus préférentiellement encore de l'ordre de 1 bar, pendant une durée comprise entre 10 à 2000 millisecondes, de préférence entre 50 à 500 millisecondes et plus préférentiellement encore de l'ordre de 100 millisecondes.

11. Procédé selon l'une quelconque des revendications précédentes, **caractérisé en ce que** le gaz inerte est l'azote.

12. Procédé selon l'une quelconque des revendications précédentes, **caractérisé en ce que** le ratio vitaminique individuel est supérieur ou égal à 98,6%.

13. Procédé selon l'une quelconque des revendications précédentes, **caractérisé en ce que** le ratio vitaminique global est supérieur ou égal à 99,3%.

14. Flacon pressurisé comprenant un produit sirupeux susceptible d'être obtenu directement par le procédé tel que défini selon la revendication 1.

15. Flacon pressurisé comprenant un produit sirupeux selon la revendication 14, **caractérisé en ce que** le ratio vitaminique global dudit produit sirupeux est supérieur ou égal à 99,3%.

## Patentansprüche

1. Verfahren zur Herstellung eines Sirupproduktes mit Vitaminen, wobei das Verfahren die folgenden Schritte umfasst:
a) die Inertisierung aller im Verfahren verwendeten Vorrichtungen
b) das Einführen eines Zucker- und/oder Zuckerderivatsirups in die Vorrichtungen, gewählt aus löslichen Fasern auf Dextrinbasis und Polyole, sowie das Einführen von Vitaminen in inerter Atmosphäre;
c) optional die Hinzugabe von dehydrierter und sauerstofffreier Glukose in inerter Atmosphäre;
d) die Desoxygenierung des so gebildeten Sirupproduktes;
e) die Konfektionierung des Sirupproduktes in inerter Atmosphäre.

2. Verfahren nach Anspruch 1, **dadurch gekennzeichnet, dass** die Einführung von Vitaminen in inerter Atmosphäre sequentiell durchgeführt wird.

3. Verfahren nach einem der vorhergehenden Ansprüche, **dadurch gekennzeichnet, dass** der Schritt des Einführens von Vitaminen ferner die Einführung eines oberflächenaktiven Mittels chemischen oder natürlichen Ursprungs umfasst, welches gewählt ist aus der Liste von Gelatinen, Pektinen, Casein, Gummi arabicum oder Galactomannanen sowie von zuvor gereinigtem und sauerstofffreiem Wasser.

4. Verfahren nach einem der vorhergehenden Ansprüche, **dadurch gekennzeichnet, dass** es zwischen den Schritten der Desoxygenierung und der Konfektionierung des Sirupproduktes einen Zwischenschritt des Speicherns des Sirupproduktes in einem Behälter in inerter Atmosphäre und unter Rühren umfasst.

5. Verfahren nach einem der vorhergehenden Ansprüche, **dadurch gekennzeichnet, dass** die Inertisierung der Atmosphäre in den im Verfahren verwendeten Vorrichtungen vor der Einführung eines beliebigen Rohstoffs durchgeführt wird, und durch Durchperlen eines inerten Gases mit einem Durchsatz von 0,1 bis 20 Nm³/h, vorzugsweise von 2 bis 8 Nm³/h und stärker bevorzugt in der Größenordnung von 4,5 Nm³/h und einem Druck von 0,1 bis 1 bar, bevorzugt von 0,3 bis 0,7 bar und noch stärker bevorzugt in der Größenordnung von 0,45 bar erhalten wird.

6. Verfahren nach einem der vorhergehenden Ansprüche, **dadurch gekennzeichnet, dass** der dehydrierten Glukose der Sauerstoff mittels eines Systems des Transfers von dehydriertem Zucker unter permanenter Desoxygenierung entzogen wird, wobei das System dann die Einführung der Glukose in den Behälter erlaubt.

7. Verfahren nach einem der vorhergehenden Ansprüche, **dadurch gekennzeichnet, dass** der Schritt der Desoxygenierung des Sirupproduktes in einem statischen Mischer durchgeführt wird, der mit einem Durchsatz eines inerten Gases von 0,1 bis 20 Nm³/h, vorzugsweise von 1 bis 10 Nm³/h und stärker bevorzugt in der Größenordnung von 8 Nm³/h und einem Druck von 0,1 bis 12 bar, bevorzugt von 0,2 bis 5 bar und noch stärker bevorzugt in der Größenordnung von 0,45 bar betrieben wird.

8. Verfahren nach einem der vorhergehenden Ansprüche, **dadurch gekennzeichnet, dass** die in dem Sirupprodukt nach dem Schritt der Desoxygenierung aufgelöste Sauerstoffmenge einen Wert von 0 bis 0,4 ppm, vorzugsweise von 0 bis 0,2 ppm und noch stärker bevorzugt von 0 bis 0,1 ppm aufweist.

9. Verfahren nach einem der vorhergehenden Ansprüche, **dadurch gekennzeichnet, dass** die Konfektionierung des Sirupproduktes mittels eines Dosierungssystems durchgeführt wird.

10. Verfahren nach einem der vorhergehenden Ansprüche, **dadurch gekennzeichnet, dass** die Flaschen, in denen das Sirupprodukt konfektioniert wird, vor ihrem Abfüllen durch Injektion eines inerten Gases mit einem Druck von 0,1 bis 5 bar, bevorzugt von 0,5 bis 2 bar und noch stärker bevorzugt in der Größenordnung von 1 bar über eine Zeitdauer von 10 bis 2000 Millisekunden, bevorzugt von 50 bis 500 Millisekunden und noch stärker bevorzugt in der Größenordnung von 100 Millisekunden inert gemacht werden.

11. Verfahren nach einem der vorhergehenden Ansprüche, **dadurch gekennzeichnet, dass** das inerte Gas Stickstoff ist.

12. Verfahren nach einem der vorhergehenden Ansprüche, **dadurch gekennzeichnet, dass** der individuelle Vitaminanteil größer als oder gleich 98,6 % ist.

13. Verfahren nach einem der vorhergehenden Ansprüche, **dadurch gekennzeichnet, dass** der allgemeine Vitaminanteil größer als oder gleich 99,3 % ist.

14. Flasche unter Druck, welche ein Sirupprodukt umfasst, das direkt durch das in Anspruch 1 beschriebene Verfahren erhalten werden kann.

15. Flasche unter Druck, welche ein Sirupprodukt nach Anspruch 14 umfasst, **dadurch gekennzeichnet, dass** der allgemeine Vitaminanteil des Sirupproduktes größer als oder gleich 99,3 % ist.

## Claims

1. A process for preparing a syrupy product comprising vitamins, said process comprising the following steps:
a) inerting all the equipment used in the process;
b) introducing, into said equipment, a sugar syrup and/or a sugar derivative chosen from chosen dextrin-based soluble fibers and polyols and introducing the vitamins under an inert atmosphere;
c) optionally adding dehydrated and deoxygenated glucose under an inert atmosphere;
d) deoxygenating the syrupy product thus formed;
e) packaging the syrupy product under an inert atmosphere.

2. The process according to claim 1, **characterized in that** the introduction of the vitamins under an inert atmosphere is carried out sequentially.

3. The process according to either one of the preceding claims, **characterized in that** the step of introducing the vitamins also comprises introducing a surfactant of chemical or natural origin chosen from the list of gelatins, pectins, casein, gum arabics or galactomannans, and also water purified and deoxygenated beforehand.

4. The process according to any one of the preceding claims, **characterized in that** it comprises an intermediate step of storing the syrupy product in a tank under an inert atmosphere and with stirring between the steps of deoxygenating and of packaging the syrupy product.

5. The process according to any one of the preceding claims, **characterized in that** the inerting of the atmosphere is carried out in the equipment used in the process before the introduction of any raw material, and is maintained by bubbling an inert gas with a flow rate ranging from 0.1 to 20 Nm³/h, preferably from 2 to 8 Nm³/h and more preferentially still of about 4.5 Nm³/h, and a pressure ranging from 0.1 to 1 bar, preferably from 0.3 to 0.7 bar and more preferentially still of about 0.45 bar.

6. The process according to any one of the preceding claims, **characterized in that** the dehydrated glucose is deoxygenated by means of a system for transferring dehydrated sugar placed under constant deoxygenation, said system subsequently making it possible to introduce the glucose into the tank.

7. The process according to any one of the preceding claims, **characterized in that** the step of deoxygenating the syrupy product is carried out in a static mixer operated with an inert gas flow rate ranging from 0.1 to 20 Nm³/h, preferably from 1 to 10 Nm³/h and more preferentially still of about 8 Nm³/h, and a pressure ranging from 0.1 to 12 bar, preferably from 0.2 to 5 bar and more preferentially still of about 0.45 bar.

8. The process according to any one of the preceding claims, **characterized in that** the dissolved oxygen content in the syrupy product after the deoxygenating step is a value ranging from 0 to 0.4 ppm, preferably from 0 to 0.2 ppm and more preferentially still from 0 to 0.1 ppm.

9. The process according to any one of the preceding claims, **characterized in that** the packaging of the syrupy product is carried out by means of a metering system.

10. The process according to any one of the preceding claims, **characterized in that** the bottles in which the syrupy product is packaged are inerted before they are filled, by injection of an inert gas with a pressure ranging from 0.1 to 5 bar, preferably from 0.5 to 2 bar and more preferentially still of about 1 bar, for a period of time of between 10 and 2000 milliseconds, preferably between 50 and 500 milliseconds and more preferentially still of about 100 milliseconds.

11. The process according to any one of the preceding claims, **characterized in that** the inert gas is nitrogen.

12. The process according to any one of the preceding claims, **characterized in that** the individual vitamin ratio is greater than or equal to 98.6%.

13. The process according to any one of the preceding claims, **characterized in that** the overall vitamin ratio is greater than or equal to 99.3%.

14. A pressurized bottle comprising a syrupy product directly obtainable by means of the process as defined in claim 1.

15. The pressurized bottle comprising a syrupy product according to claim 14, **characterized in that** the overall vitamin ratio of said syrupy product is greater than or equal to 99.3%.
